# EUROPEAN PATENT SPECIFICATION

(11) **EP 2 877 195 B1**
(45) Date of publication and mention of the grant of the patent: **21.03.2018**
(21) Application number: 13822996.8
(22) Date of filing: 26.07.2013
(51) Int. Cl.: A61K 38/08, A61K 9/06, A61P 27/02

(54) **METHOD OF TREATING OPTIC NERVE DAMAGE, OPHTHALMIC ISCHEMIA OR OPHTHALMIC REPERFUSION INJURY**
VERFAHREN ZUR BEHANDLUNG VON SEHNERVENSCHÄDEN, OPHTHALMISCHER ISCHÄMIE ODER OPHTHALMISCHEN REPERFUSIONSSCHÄDEN
PROCÉDÉ DE TRAITEMENT D'UNE LÉSION DU NERF OPTIQUE, D'UNE ISCHÉMIE OPHTALMIQUE OU D'UNE LÉSION DE REPERFUSION OPHTALMIQUE

(30) Priority: 27.07.2012 NZ 60151212
(43) Date of publication of application: 03.06.2015
(73) Proprietor: Curonz Holdings Company Limited, Auckland (NZ)
(72) Inventor: SIEG, Frank, Mangawhai 0975 (NZ)
(74) Representative: Kremer, Simon Mark
(86) International application number: PCT/NZ2013/000132
(87) International publication number: WO 2014/017927

(56) References cited:
- WO-A1-2009/051844
- WO-A2-2005/042561
- WO-A2-2006/121926
- WO-A2-2007/011595
- WO-A2-2012/102625
- WO-A2-2012/105854
- , 16 February 2006 (2006-02-16), XP055220101, Retrieved from the Internet: URL:http://www.asx.com.au/asxpdf/20060216/ pdf/3vg7vcls6325d.pdf [retrieved on 2015-10-12]
- GORBA ET AL.: 'Neural regeneration protein is a novel chemoattractive and neuronal survival-promoting factor' EXPERIMENTAL CELL RESEARCH vol. 312, no. 16, 2006, pages 3060 - 3074, XP024945194
- BENOWITZ ET AL.: 'Optic nerve regeneration' ARCHIVES OF OPHTHALMOLOGY vol. 128, no. 8, 2010, pages 1059 - 1064, XP055190905
- ZHANG ET AL.: 'Ophthalmic drug discovery: novel targets and mechanisms for retinal diseases and glaucoma' NATURE REVIEWS DRUG DISCOVERY vol. LL, no. 7, 15 June 2012, pages 541 - 559, XP055060459

## Description

### FIELD OF INVENTION

The invention relates to peptides for use in a method of treating optic nerve damage, ophthalmic ischemia or ophthalmic reperfusion injury.

### BACKGROUND ART

Progressive loss of retinal ganglion cells (RGCs) is a hallmark of traumatic or glaucoma-like injury of the optic nerve (Soto et al., 2008). Apart from the initial primary injury to retinal neurons caused by the neurodegenerative disease process there is a secondary apoptotic process assumed that is mediated by the elevation of excitotoxins like extracellular glutamate causing further damage to the retina (Prokosch et al., 2010). There is a strong need to identify neuroprotective substances that will be therapeutically effective in a clinical relevant setting. Brimonidine, an alpha-2A-adrenergic receptor agonist has been shown to be neuroprotective in formulations either topically or intraperitoneal (IP) applied within various ischemia-related optic nerve injury animal models (Weber et al., 2007; Yoles et al., 1999; Levkovitch-Verbin et al., 2000 and Loengren et al., 2006). It is known that Brimonidine is only active when administered in prophylactic fashion within *in vivo* rodent models of optic nerve ischemia but loses its effect dramatically in regard to promotion of retinal ganglion cell (RGC) survival even when applied minutes after *ex vivo* performed injuries of retinal tissue (Prokosch et al., 2010). So far, Brimodine has shown promising neuroprotective activities in rodent models of optic nerve damage but failed to do so when used in comparable human ophthalmic diseases.

WO 2009/051844 A1 discloses the use of neural regeneration peptides for the treatment of neurological disorders of the brain, particularly experimental autoimmune encephalomyelitis, multiple sclerosis, amyotrophic lateral sclerosis and toxic injury to neural cells. This document also discloses administration of these peptides by subcutaneous injections and direct administration to the CNS.

It is an object of this invention to provide a method for use in such treatments comprising a neural regeneration peptide or to at least to provide a useful choice.

### STATEMENT OF INVENTION

In a first aspect the invention provides a peptide comprising the sequence:
GlyArgArgAlaAlaProGlyArgAibGlyGly (SEQ ID NO:1) for use in a method of treating or preventing (i) optic nerve damage; (ii) an ophthalmic reperfusion injury; or (iii) ophthalmic ischemia; which method comprises administering an effective amount of the peptide, in the form of a medicament adapted for topical ophthalmic administration, to an eye of a subject in need thereof.

In one embodiment the peptide consists of the 11 amino acid residue sequence:
GlyArgArgAlaAlaProGlyArgAibGlyGly
or a derivatives thereof obtained by amidation, acylation, alkylation, carboxylation, glycosylation, phosphorylation, prenylation, salification, sulfation, or a combination thereof. One such ophthalmically acceptable derivative includes the sequence wherein the C-terminus of the peptide is amidated to give: GlyArgArgAlaAlaProGlyArgAibGlyGly-NH₂(SEQ ID NO:2).

In one embodiment the administration step to the subject is by way of one or more topically applied eye drops. In another embodiment the administration step to the subject's eye may be by way of administration of a cream or an ointment. In another embodiment the administration step to the subject's eye is by way of a liquid drop preparation applied to the conjunctival sac of the eye of the subject. Disclosed herein are peptides for use whereby the administration step to the subject's eye is by way of an intravitreal injection.

In one embodiment the peptide for use includes the step of administering an effective amount of a peptide comprising the sequence:
GlyArgArgAlaAlaProGlyArgAibGlyGly (SEQ ID NO:1)or
GlyArgArgAlaAlaProGlyArgAibGlyGly-NH₂(SEQ ID NO:2)
to an eye of the subject in need thereof on an at least once a day basis.

In one embodiment the peptide for use includes the step of administering an effective amount of a peptide comprising the sequence:
GlyArgArgAlaAlaProGlyArgAibGlyGly (SEQ ID NO:1)
to an eye of the subject in need thereof on an at least once a day basis.

In one embodiment the peptides for use defined above include the step of administering the peptide on an at least twice a day basis.

In one embodiment the subject is selected from the group consisting of: humans and companion animals.

In some embodiments, the peptide for use is for use in a method of treating or preventing optic nerve damage. In other embodiments, the peptide is for use in a method of treating or preventing ophthalmic reperfusion injury. In other embodiments, the peptide for use is for use in a method of treating or preventing ophthalmic ischemia.

It will be understood from the following description that the effective amount of the peptide of SEQ ID NO:1 or SEQ ID NO: 2 is indicated to be in the range of 2-20 nanogram dose amounts when administered in rodent models of optic nerve injury and in the range of 20-200 nanogram dose amounts when administered to larger subjects such as dogs or humans.

In the description and claims of this specification the following acronyms, terms and phrases have the meaning provided:
"Effective amount" means an amount effective to treat or prevent optic nerve damage; an ophthalmic reperfusion injury; or ophthalmic ischemia in a given subject.

"Functionally similar amino acid" means an amino acid with similar properties according to the following groupings:
Neutral-weakly hydrophobic (Ala, Gly, Pro, Ser, Thr)
Hydrophilic-Acid Amine (Asn, Asp, Gin, Glu)
Hydrophilic-Basic (Arg, His, Lys)
Hydrophobic (Ile, Met, Leu, Val)
Hydrophobic-Aromatic (Phe, Trp, Tyr)
Cross-linking (Cys)

"Ophthalmologically acceptable derivatives" means derivatives of the peptide defined in SEQ ID NO:1 obtained by amidation, acylation, alkylation, carboxylation, glycosylation, phosphorylation, prenylation, salification, sulfation, or a combination thereof, that are suitable for inclusion in a composition for administration to the eye.

"Ophthalmologically acceptable excipients" means excipients selected from stabilizing agents, surfactants, buffering agents, chelating agents, viscosity agents, tonicity agents and preservative agents that are suitable for inclusion in a composition for administration to the eye.

In the description and claims of this specification the nucleotides and amino acids of biosequences (nucleic acids and peptides) are identified in accordance with Tables 1 to 4 of Annex C, Appendix 2 of the PCT Administrative Instructions (as in force from January 1, 2010).

The invention will now be described with reference to embodiments or examples and the figures of the accompanying drawings pages.

### BRIEF DESCRIPTION OF DRAWINGS

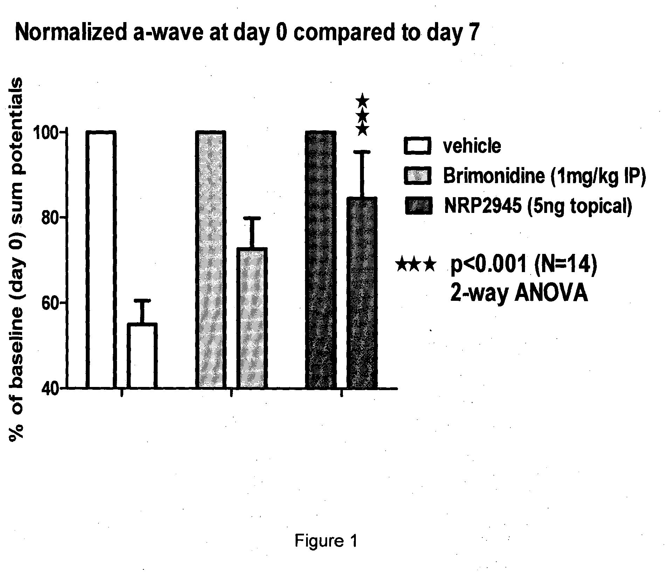

Figure 1. Shows a plot of a-wave amplitude results wherein SEQ ID NO: 1 also known as NRP2945 was administered as an eye drop (twice daily) in one group of rats starting at 30min after optic nerve ligation / reperfusion injury compared to prophylatically administered Brimonidine to another group of rats.

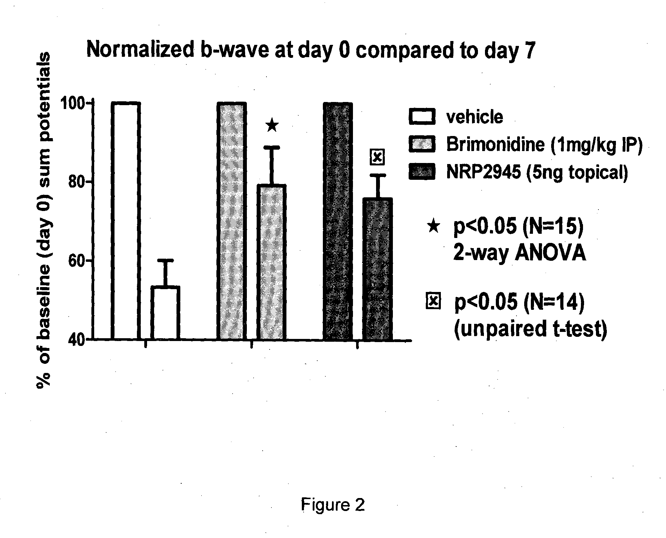

Figure 2. Shows a plot of b-wave results where SEQ ID NO: 1, also known as NRP2945, was administered as an eye drop (twice daily) in one group of rats starting at 30min after optic nerve ligation / reperfusion injury compared to the prophylactically administered Brimonidine to another group of rats.

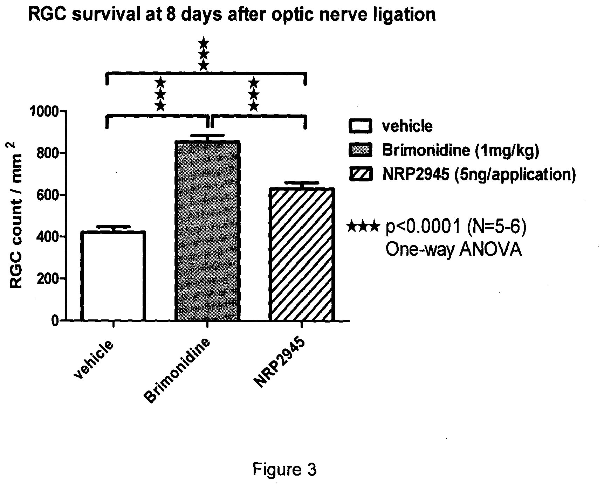

Figure 3. Shows a plot of retinal ganglion cells (RCG) survival after SEQ ID NO: 1 also known as NRP2945 was applied 16 x times (twice daily)to a first group of rats starting at 30min after optic nerve ligation/ reperfusion as an eye drop to the cornea of each restrained rat compared to a group of rats rescued by prophylactically applied Brimonidine.

Further aspects of the invention will become apparent with reference to the accompanying Figures and Examples described below:

### Example 1: NRP2945 efficacy in a rat model of optic nerve ligation

### Animals

Male Long-Evans rats (aged P50) were housed for up to 7 days before the start of experimentation and were monitored for signs of ill health. Animals displaying ocular abnormalities were excluded from the study. Every rat was monitored for body weight daily.

### Grouping of animals for optic nerve ligation study

Animals were assessed by measuring a baseline electro-retinogram (ERG) at day 0 just before injury in order to normalize all rats in respect of their b-wave amplitude value and group them into three groups as detailed below and as shown in Table 1:
Group 1 received 5ng of NRP2945 reconstituted in saline twice daily as an eye drop (starting with 30min after surgery-reperfusion). Only one eye per animal received the injury and drug treatment, while the non-injured eye served as control.
Group 2 received one dose of physiological saline (intra-vitreal route at 30min after surgery-reperfusion);
Group 3 received the adrenergic α-type 2 agonist brimonidine in prophylactic fashion at a concentration of 1mg/kg (IP-route at 1 hr before injury).

**Table 1**

| Group No. | **Treatment** | **Dose** | **Route of admin. (volume)** | **Time of admin.** | **Number of animals** |
|---|---|---|---|---|---|
| 1 | NRP2945 | 5 ng/eye | Right eye topical instillation (12.5µl) | Day 0 to Day 7: twice daily | 14 |
| 2 | Vehicle (Saline) | - | Right eye intravitreal route (5µl) | Day 0 (just after ischemia) | 16 |
| 3 | Brimonidine (0.2% w/v) | 1 mg/kg | Intraperitoneal 0.5 ml/kg | Day 0: 1h before ischemia | 15 |

### ERG evaluation and measurements

ERG measurements were recorded before ischemia (baseline) and 7 days after reperfusion on both eyes in dark-adapted animals. The latency times (for a- and b-wave) and the a-wave and b-wave amplitudes (µV) was measured for each ERG; the a-wave and b-wave amplitudes was expressed as a percentage of the baseline value obtained before ischemia. 15 min before measurement 10 µl Mydriaticum® (0.5% tropicamide) was instilled for pupillary dilatation.

### ERG parameters:

Color: white maximum.
Maximum intensity: 2.6 cd.s/m2 (0dB); Duration 0.24 ms; 1 flash
Filter: 50 Hz.
Impedence Threshold: 90 kΩ.

### Method of optic nerve ligation

Animals were anesthetized by an intramuscular injection of a mix of 2mg/kg xylazine and 2mg/kg ketamine. For the vascular ligation model right eyes underwent a temporal orbitectomy combined with periorbital stripping. The globe remained in the orbit and was completely isolated on a pedicle consisting of the optic nerve, ophthalmociliary arteries and the venous outflow. A ligation placed around the pedicle initiated the global ocular ischemia when the ligation was tightened. Ischemia was maintained for 45 minutes. The reperfusion period was initiated by the release of the ligation.

### Study Termination and RGC Evaluation

At the end of the study, the animals were euthanized by intraperitoneal injection of overdosed pentobarbital. After euthanasia at day 8 after optic nerve ligation injury, the retinae of both eyes of N=6 animals per cohort were fixed in formalin 4% (1 h at room temperature), dissected and flat-mounted. The flat-mounted preparation was incubated with an Alexa 594 conjugated anti-BRN3A (Brain-specific homeobox/POU domain protein 3A, Chemicon, cat #mAb1585) to visualize the Retinal Ganglion Cells (RGC). Fluorescence was assessed by an Apotome microscope at magnification x 20 (Zeiss) within twelve randomly selected respective microscopic fields per subject. The number of surviving RGC was determined with Axio Vision 4.2 software in the respective retinae areas.

### Results

As shown in Figure 1, wherein SEQ ID NO: 1 also known as NRP2945, was administered as an eye drop (twice daily) starting at 30min after optic nerve ligation / reperfusion injury, a recovery of 84.5% of the initial a-wave on day 7 after injury was measured. In comparison, the prophylactically administered Brimonidine led to a 72.6% recovery, while the vehicle conditions only led to a 57.0% recovery of the initial a-wave amplitude.

As shown in Figure 2, SEQ ID NO: 1 also known as NRP2945 was administered as an eye drop (twice daily) starting at 30min after optic nerve ligation / reperfusion injury, which led to a recovery of 75.8% of the initial b-wave on day 7 after injury. In comparison, the prophylactically administered Brimonidine led to 79.1% recovery, while the vehicle conditions only led to a 58.4% recovery of the initial b-wave amplitude.

As shown in Figure 3, SEQ ID NO: 1 also known as NRP2945 was applied 16 x times (twice daily) starting at 30min after optic nerve ligation/ reperfusion as an eyedrop to the cornea of a restrained rat. Animals were sacrificed at day 8 and retinae were analysed for BrN3A (Brain-specific homeobox/POU domain protein 3A) protein expression patterns that are specific for retinal ganglion cells (RGCs). 12 fields per retina were evaluated.

The mean RGC density in retina of non-ischemic eyes in this assay was 2158 RGCs /mm2 (n=18). RGC density decreased to 421.0 ± 25.9 RGCs/mm2 at 8 days after ischemia (19.6% compared to non-ischemic contralateral eyes) in saline treated group). NRP2945 cohorts showed 629.8 ± 30.3 cells/mm2 (p<0.001, n = 6) at 8 days after injury.

NRP2945 rescued 29.2% of total RGCs compared to 39.6% (855.5 ± 30.7 RGCs/mm2 with p<0.001, n=5) of cells rescued by prophylactically applied Brimonidine. All differences between the respective cohorts are highly statistically significant.

Although the invention has been described with reference to an embodiment or example it should be appreciated that variations and modifications may be made to this embodiment or example without departing from the scope of the invention.

Where known equivalents exist to specific features, such equivalents are incorporated as if specifically referred to in this specification.

In particular, it is anticipated that functionally similar peptide sequences may be obtained by substitution of one or more amino acids of the biosequence with a functionally similar amino acid. It is suggested that the functionality of similar peptide sequences may be confirmed without undue additional experimentation by use of the method disclosed in this specification.

### REFERENCES

Soto, I., Oglesby, E., Buckingham, B.P., Son, J.L., Roberson, E.D.O., Steele, M.R., Inman, D.M., Vetter, M.L., Horner, P.J. and Marsh-Armstrong, N. (2008). Retinal Ganglion Cells down-regulate gene expression and lose their axons within the optic nerve head in a mouse glaucoma model. J Neurosci 28: 548-561.
Prokosch, V., Panagis, L., Volk, G.F., Dermon, C. and Thanos, S. (2010). α-2-adrenergic receptors and their core involvement in the process of axonal growth in retinal explants. Invest. Ophthalmology 51: 6688-6699.
Weber, B., Steinfath, M., Scholz, J. and Bein, B. (2007). Neuroprotective effects of α-2-adrenergic receptor agonists. Drug News Perspect 20: 149-154.
Yoles, E., Wheeler, L.A. and Schwartz, M. (1999). α-2-adrenoreceptor agonists are neuroprotective in a rat model of optic nerve degeneration. Invest Ophthalmol Vis Sci 40: 65-73.
Levkovitch-Verbin, H., Harris-Cerruti, C., Groner, Y., Wheeler, L.A., Schwartz, M. and Yoles, E. (2000). RGC death in mice after optic nerve crush injury: oxidative stress and neuroprotection. Invest Ophthalmol Vis Sci 41: 4169-4174.
Loenngren, U., Naepaenkangas, U., Lafuente, M., Mayor, S., Lindqvist, N., Vidal-Sanz, M. and Hallbook, F. (2006). The growth factor response in ischemic rat retina and superior colliculus after brimonidine pre-treatment. Brain Res Bulletin 71: 208-218

## Claims

1. A peptide comprising the sequence
GlyArgArgAlaAlaProGlyArgAibGlyGly (SEQ ID NO:1.)
for use in a method of treating or preventing
(i) optic nerve damage;
(ii) an ophthalmic reperfusion injury; or
(iii) ophthalmic ischemia;
which method comprises administering an effective amount of the peptide, in the form of a medicament adapted for topical ophthalmic administration, to an eye of a subject in need thereof.

2. The peptide for use as claimed in claim 1 wherein the peptide consists of the 11 amino acid residue sequence:
GlyArgArgAlaAlaProGlyArgAibGlyGly
or a derivative thereof obtained by amidation, acylation, alkylation, carboxylation, glycosylation, phosphorylation, prenylation, salification, sulfation, or a combination thereof.

3. The peptide for use as claimed in claim 2 wherein the peptide consists of an ophthalmically acceptable derivative wherein the C-terminus of the peptide is amidated to give: GlyArgArgAlaAlaProGlyArgAibGlyGly-NH₂(SEQ ID NO:2).

4. The peptide for use as claimed in any one of claims 1 to 3 wherein the administration step to the subject is by way of one or more topically applied eye drops.

5. The peptide for use as claimed in any one of claims 1 to 3 wherein the administration step to the subject's eye is by way of administration of a cream or an ointment.

6. The peptide for use as claimed in any one of claims 1 to 3 wherein the administration step to the subject's eye is by way of a liquid formulation applied to the conjunctival sac of the eye of the subject.

7. The peptide for use of any one of claims 1 to 6 wherein an effective amount of a peptide comprising the sequence:
GlyArgArgAlaAlaProGlyArgAibGlyGly (SEQ ID NO:1) or
GlyArgArgAlaAlaProGlyArgAibGlyGly-NH₂(SEQ ID NO:2)
is administered to an eye of the subject in need thereof on an at least once a day basis.

8. The peptide for use of claim 7 wherein the peptide is administered to an eye of the subject in need thereof on an at least twice a day basis.

9. The peptide for use of any one of claims 1 to 6 wherein said method includes the step of administering an effective amount of a peptide comprising the sequence:
GlyArgArgAlaAlaProGlyArgAibGlyGly (SEQ ID NO:1)
to an eye of the subject in need thereof on an at least once a day basis.

10. The peptide for use of claim 9 wherein the peptide is administered to an eye of the subject in need thereof on an at least twice a day basis.

11. The peptide for use of any one of claims 1 to 10 wherein the subject is selected from the group consisting of: humans and companion animals.

12. The peptide for use of any one of claims 1 to 11, which is for use in a method of treating or preventing optic nerve damage.

13. The peptide for use of any one of claims 1 to 11, which is for use in a method of treating or preventing ophthalmic reperfusion injury.

14. The peptide for use of any one of claims 1 to 11, which is for use in a method of treating or preventing ophthalmic ischemia.

## Patentansprüche

1. Peptid, umfassend die Sequenz
GlyArgArgAlaAlaProGlyArgAibGlyGly (Seq.-ID Nr. 1),
zur Verwendung in einem Verfahren zur Behandlung oder Prävention
(i) eines Sehnervschadens;
(ii) einer ophthalmischen Reperfusionsverletzung; oder
(iii) ophthalmischer Ischämie;
wobei das Verfahren die Verabreichung einer wirksamen Menge des Peptids in Form eines für topische ophthalmische Verabreichung adaptierten Medikaments an ein Auge eines Individuums, das dessen bedarf, umfasst.

2. Peptid zur Verwendung nach Anspruch 1, wobei das Peptid aus der 11-Aminosäurerestesequenz
GlyArgArgAlaAlaProGlyArgAibGlyGly
oder einem Derivat davon besteht, die/das durch Amidierung, Acylierung, Alkylierung, Carboxylierung, Glykosylierung, Phosphorylierung, Prenylierung, Salzbildung, Sulfatierung oder einer Kombination davon erhalten wurde.

3. Peptid zur Verwendung nach Anspruch 2, wobei das Peptid aus einem ophthalmisch annehmbaren Derivat besteht, wobei der C-Terminus des Peptids amidiert ist, um Folgendes zu erhalten: GlyArgArgAlaAlaProGlyArgAibGlyGly-NH₂ (Seq.-ID Nr. 2).

4. Peptid zur Verwendung nach einem der Ansprüche 1 bis 3, wobei der Verabreichungsschritt an das Individuum mittels eines oder mehrerer topisch angewendeter Augentropfen erfolgt.

5. Peptid zur Verwendung nach einem der Ansprüche 1 bis 3, wobei der Verabreichungsschritt an das Auge des Individuums mittels Verabreichung einer Creme oder Salbe erfolgt.

6. Peptid zur Verwendung nach einem der Ansprüche 1 bis 3, wobei der Verabreichungsschritt an das Auge des Individuums mittels einer flüssigen Formulierung erfolgt, die auf den Bindehautsack des Auges des Individuums aufgebracht wird.

7. Peptid zur Verwendung nach einem der Ansprüche 1 bis 6, wobei eine wirksame Menge eines Peptids, das die folgende Sequenz umfasst:
GlyArgArgAlaAlaProGlyArgAibGlyGly (Seq.-ID Nr. 1) oder
GlyArgArgAlaAlaProGlyArgAibGlyGly-NH₂ (Seq.-ID Nr. 2),
an ein Auge des Individuums, das dessen bedarf, zumindest einmal täglich verabreicht wird.

8. Peptid zur Verwendung nach Anspruch 7, wobei das Peptid an ein Auge des Individuums, das dessen bedarf, zumindest zweimal täglich verabreicht wird.

9. Peptid zur Verwendung nach einem der Ansprüche 1 bis 6, wobei das Verfahren den Schritt des Verabreichens einer wirksamen Menge eines Peptids, das die folgende Sequenz umfasst:
GlyArgArgAlaAlaProGlyArgAibGlyGly (Seq.-ID Nr. 1),
an ein Auge des Individuums, das dessen bedarf, zumindest einmal täglich umfasst.

10. Peptid zur Verwendung nach Anspruch 9, wobei das Peptid an ein Auge des Individuums, das dessen bedarf, zumindest zweimal täglich verabreicht wird.

11. Peptid zur Verwendung nach einem der Ansprüche 1 bis 10, wobei das Individuum aus der aus Menschen und Haustieren bestehenden Gruppe ausgewählt ist.

12. Peptid zur Verwendung nach einem der Ansprüche 1 bis 11 zur Verwendung in einem Verfahren zur Behandlung oder Prävention eines Sehnervschadens.

13. Peptid zur Verwendung nach einem der Ansprüche 1 bis 11 zur Verwendung in einem Verfahren zur Behandlung oder Prävention einer ophthalmischen Reperfusionsverletzung.

14. Peptid zur Verwendung nach einem der Ansprüche 1 bis 11 zur Verwendung in einem Verfahren zur Behandlung oder Prävention einer ophthalmischen Ischämie.

## Revendications

1. Peptide comprenant la séquence
GlyArgArgAlaAlaProGlyArgAibGlyGly (SEQ ID NO:1.)
pour une utilisation dans un procédé de traitement ou de prévention
(i) d'une lésion du nerf optique ;
(ii) d'une lésion de reperfusion ophtalmique ; ou
(iii) de l'ischémie ophtalmique ;
lequel procédé comprend l'administration d'une quantité efficace du peptide, sous la forme d'un médicament adapté pour une administration ophtalmique topique, à un oeil d'un sujet en ayant besoin.

2. Peptide pour une utilisation selon la revendication 1, dans lequel le peptide consiste en la séquence de 11 résidus d'acides aminés :
GlyArgArgAlaAlaProGlyArgAibGlyGly
ou un dérivé de celui-ci obtenu par amidation, acylation, alkylation, carboxylation, glycosylation, phosphorylation, prénylation, salification, sulfatation, ou une combinaison de ceux-ci.

3. Peptide pour une utilisation selon la revendication 2, dans lequel le peptide est constitué d'un dérivé ophtalmiquement acceptable dans lequel l'extrémité C-terminale du peptide est amidée pour donner :
GlyArgArgAlaAlaProGlyArgAibGlyGly-NH₂ (SEQ ID NO:2).

4. Peptide pour une utilisation selon l'une quelconque des revendication 1 à 3, dans lequel l'étape d'administration au sujet s'effectue au moyen d'une ou de plusieurs gouttes ophtalmiques appliquées localement.

5. Peptide pour une utilisation selon l'une quelconque des revendication 1 à 3, dans lequel l'étape d'administration à l'oeil du sujet consiste à administrer une crème ou une pommade.

6. Peptide pour une utilisation selon l'une quelconque des revendication 1 à 3, dans lequel l'étape d'administration à l'oeil du sujet s'effectue au moyen d'une formulation liquide appliquée au sac conjonctival de l'oeil du sujet.

7. Peptide pour une utilisation selon l'une quelconque des revendication 1 à 6 dans lequel une quantité efficace d'un peptide comprenant la séquence :
GlyArgArgAlaAlaProGlyArgAibGlyGly (SEQ ID NO:1) ou
GlyArgArgAlaAlaProGlyArgAibGlyGly-NH₂ (SEQ ID NO:2)
est administrée à une oeil du sujet en ayant besoin au moins une fois par jour.

8. Peptide pour une utilisation selon la revendication 7 dans lequel le peptide est administré à un oeil du sujet en ayant besoin au moins deux fois par jour.

9. Peptide pour une utilisation selon l'une quelconque des revendication 1 à 6, dans lequel ledit procédé comprend l'étape d'administration d'une quantité efficace d'un peptide comprenant la séquence :
GlyArgArgAlaAlaProGlyArgAibGlyGly (SEQ ID NO:1)
à un oeil du sujet en ayant besoin au moins une fois par jour.

10. Peptide pour une utilisation selon la revendication 9, dans lequel le peptide est administré à un oeil du sujet en ayant besoin au moins deux fois par jour.

11. Peptide pour une utilisation selon l'une quelconque des revendication 1 à 10, dans lequel le sujet est choisi dans le groupe constitué par : des humains et des animaux de compagnie.

12. Peptide pour une utilisation selon l'une quelconque des revendication 1 à 11, qui est destiné à une utilisation dans un procédé de traitement ou de prévention d'une lésion du nerf optique.

13. Peptide pour une utilisation selon l'une quelconque des revendication 1 à 11, qui est destiné à une utilisation dans un procédé de traitement ou de prévention d'une lésion de reperfusion ophtalmique.

14. Peptide pour une utilisation selon l'une quelconque des revendication 1 à 11, qui est destiné à une utilisation dans un procédé de traitement ou de prévention de l'ischémie ophtalmique.


### SEQUENCE LISTING

<110> Curonz Holdings Company Limited
<120> METHOD OF TREATING OPTIC NERVE DAMAGE, OPHTHALMIC ISCHEMIA OR OPHTHALIMIC REPERFUSION INJURY
<130> 441 WO1
<160> 2
<170> Patentln version 3.5
<210> 1
   <211> 11
   <212> PRT
   <213> Human
<220>
   <221> MOD_RES
   <222> (9)..(9)
   <223> METHYLATION, Aib
<400> 1
   Gly Arg Arg Ala Ala Pro Gly Arg Ala Gly Gly
   1               5                   10
<210> 2
   <211> 11
   <212> PRT
   <213> Human
<220>
   <221> MOD_RES
   <222> (9)..(9)
   <223> METHYLATION, Aib
<220>
   <221> MOD_RES
   <222> (11)..(11)
   <223> AMIDATION
<400> 2
   Gly Arg Arg Ala Ala Pro Gly Arg Ala Gly Gly
   1 5 10